# EUROPÄISCHE PATENTANMELDUNG

(11) **EP 1 055 653 A2**
(43) Veröffentlichungstag der Anmeldung: **29.11.2000**
(21) Anmeldenummer: 00110915.6
(22) Anmeldetag: 24.05.2000
(51) Int. Cl.: C07B 37/02, C07B 41/04, C07B 43/04, C07C 41/08, C07C 209/60, C07C 253/30, C07D 207/32, C07D 209/08, C07D 233/58, C07D 235/06

(54) **Verfahren zur Herstellung von Alken-Verbindungen**

(30) Priorität: 26.05.1999 DE 19924050
(71) Anmelder: BASF AKTIENGESELLSCHAFT, 67056 Ludwigshafen (DE)
(72) Erfinder: Aiscar Bayeto, Juan Jose, Dr., 68176 Mannheim (DE); Henkelmann, Jochem, Dr., 68165 Mannheim (DE); Preiss, Thomas, Dr., 67256 Weisenheim am Sand (DE); Knochel, Paul, Prof. Dr., 81475 München (DE); Tzalis, Dimitrios, Dr., 35043 Marburg (DE); Koradin, Christopher, Dr., 35260 Stadtallendorf (DE)
(74) Vertreter: Isenbruck, Günter, Dr.

(57) **Zusammenfassung**

CsOH · H₂O wird als Katalysator bei der Alkenylierung von organischen Verbindungen, die Gruppen -NH, -OH oder >CH-Z
mit Z = CN, COOR
mit R = H oder C₁₋₆-Alkyl
enthalten, mit Ausnahme von 1,2-Aminoalkanolen, durch Umsetzung der Gruppen mit organischen Verbindungen, die eine C-C-Dreifachbindung enthalten, verwendet.

N-Methylpyrrolidon wird als Lösungsmittel verwendet.

## Beschreibung

Die Erfindung betrifft Verfahren zur Herstellung von Alken-Verbindungen durch Umsetzung von Verbindungen, die Gruppen mit abstrahierbaren Protonen aufweisen, mit Verbindungen, die eine C-C-Dreifachbindung enthalten, in Gegenwart von basischen Katalysatoren.

Die Basen-katalysierte Bildung von neuen Kohlenstoff-Kohlenstoff- oder Kohlenstoff-Heteroatom-Bindungen ist ein wichtiges Synthesewerkzeug. Besonders attraktiv in dieser Kategorie von Umsetzungen sind Additionen an Kohlenstoff-Kohlenstoff-Mehrfachbindungen, die ohne die Bildung von Seitenprodukten durchgeführt werden können und damit atomökonomisch sind. Beispielsweise ist die metall-katalysierte Addition von Alkinen an Carbonyl-Verbindungen zur Herstellung von Propargylalkoholen von technischer Bedeutung. Die Durchführung dieser Umsetzungen unter Verwendung einer stöchiometrischen Menge an Base wie Organolithium- oder Organomagnesium-Verbindungen zur Bildung eines Metallacetylid-Zwischenproduktes ist seit langem bekannt.

In W. Reppe, Liebigs Ann. Chem. 1955, 596, Seiten 1 bis 3, wird die Carbonylierung von Acetylen mit Carbonyl-Verbindungen in Gegenwart von Acetyliden der Schwermetalle der ersten Nebengruppe des Periodensystems der Elemente in THF beschrieben.

Auf den Seiten 6 bis 11 und 25 bis 38 wird beschrieben, daß die Ethinylierung von Ketonen in wäßrigen Medien unter Verwendung von Alkalihydroxiden, Erdalkalihydroxiden, Alkalicarbonaten oder tertiären Aminen als Katalysatoren durchgeführt werden kann. Zudem kann Kupferacetylid als Katalysator verwendet werden. In den Beispielen wird die Herstellung von Methylbutinol aus Aceton und Acetylen in Wasser mit Kaliumhydroxid als Katalysator erwähnt.

J. H. Babler et al., J. Org. Chem. 1996, 61, Seiten 416 bis 417, beschreiben die Alkoxid-katalysierte Addition von terminalen Alkinen an Ketone. Dabei wird die Umsetzung mit tert.-Butoxid als Katalysator in DMSO als Lösungsmittel durchgeführt.

M. Makosza, Tetrahedron Lett. 1966, Seiten 5489 bis 5492, beschreibt die Vinylierung von Phenylacetonitril-Verbindungen in Gegenwart eines Systems aus Natriumhydroxid, DMSO und Triethylbenzylammoniumchlorid.

In B. F. Kukharev et al., Russian Journal of Organic Chemistry, Vo. 29, No. 12, 1993, Seiten 2005 bis 2012, ist die nucleophile Addition von 1,2-Aminoalkanolen an Phenylacetylene beschrieben. Dabei kann neben anderen Alkalimetallhydroxiden Cäsiumhydroxid lösungsmittelfrei oder in DMSO als Lösungsmittel eingesetzt werden. Die Alkenylierung erfolgt an der Hydroxyl-Gruppe des 1,2-Aminoalkanols.

Dimethylsulfoxid (DMSO) ist für technische Verfahren unvorteilhaft, da es kostspielig und toxikologisch nicht unbedenklich sowie thermisch instabil ist.

Aufgabe der vorliegenden Erfindung ist die Bereitstellung verbesserter und allgemein verwendbarer Verfahren zur Herstellung von Alken-Verbindungen durch

Umsetzung von organischen Verbindungen, die Amino-, Hydroxyl- und/oder Nitril-Gruppen aufweisen, mit Verbindungen, die acetylenische Gruppen (-C≡C-H) aufweisen.

Die Aufgabe wird gelöst durch ein Verfahren zur Herstellung von Alken-Verbindungen der allgemeinen Formel (IV) 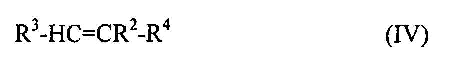 mit der Bedeutung
- R², R³: unabhängig H, NH₂ oder C₁₋₂₀-Kohlenwasserstoffrest, der ein- oder mehrfach durch C₁₋₆-Alkyl und/oder Halogen substituiert und/oder von nicht benachbarten Heteroatomen unterbrochen sein und/oder C-C-Doppelbindungen enthalten kann,
- R⁴: R⁵R⁶N-, R⁵R⁶C(Z)- oder R⁷-O- mit
- R⁵, R⁶, R⁷: unabhängig wie für R³ definiert mit Ausnahme von H, wobei R⁷-O-kein 1,2-Aminoalkanolrest sein kann,
- Z: CN, COOR mit R = H oder C₁₋₆-Alkyl,
durch Umsetzung von Verbindungen der allgemeinen Formel (V) 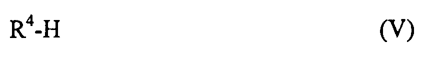 ausgenommen 1,2-Aminoalkanole,
mit Verbindungen der allgemeinen Formel (III) 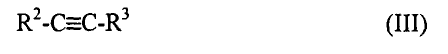 in Gegenwart eines basischen Katalysators, wobei als basischer Katalysator CsOH · H₂O eingesetzt wird und/oder die Umsetzung in einem Lösungsmittel auf der Basis von N-Methylpyrrolidon (NMP) durchgeführt wird. Bei Verwendung eines Lösungsmittels auf Basis von NMP können auch 1,2-Aminoalkanole als Verbindung der allgemeinen Formel (V) eingesetzt werden.

Es wurde erfindungsgemäß gefunden, daß die Umsetzung für eine Vielzahl von Verbindungen mit verbesserter Katalysatoraktivität und Ausbeute durchgeführt werden kann, wenn als basischer Katalysator CsOH · H₂O eingesetzt wird. Die Umsetzung kann dabei lösungsmittelfrei oder in geeigneten Lösungsmitteln erfolgen. Besonders bevorzugt ist die Umsetzung in einem Lösungsmittel auf Basis von N-Methylpyrrolidon (NMP). Der Ausdruck 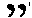 auf Basis von" bedeutet, daß auch Lösungsmittelgemische eingesetzt werden können, die im wesentlichen NMP enthalten. Beispielsweise können Mengen von bis zu 20 Gew.-%, vorzugsweise bis zu 10 Gew.-%, insbesondere bis zu 5 Gew.-% anderer Lösungsmittel toleriert werden. Damit weist das Lösungsmittel vorzugsweise einen Gehalt von mindestens 80 Gew.-%' besonders bevorzugt mindestens 90 Gew.-%, insbesondere mindestens 95 Gew.-% NMP auf. Der verbleibende Rest des Lösungsmittels ist dabei gegenüber den Umsetzungen inert. Beispiele sind DMSO und THF sowie Gemische davon. Speziell bevorzugt wird im wesentlichen oder ganz ausschließlich NMP als Lösungsmittel eingesetzt.

Die Verwendung von CsOH · H₂O ist auch im Zusammenhang mit anderen Lösungsmitteln wie DMSO, THF oder Gemischen aus THF und DMSO oder unter Verzicht auf Lösungsmittel möglich. Besonders gute Ergebnisse erzielt man jedoch bei einer Kombination von CsOH · H₂O mit NMP als Lösungsmittel.

Entsprechend ist die Umsetzung in einem Lösungsmittel auf der Basis von NMP mit einer Vielzahl basischer Katalysatoren möglich. Vorzugsweise wird die Umsetzung in Gegenwart von Alkalihydroxiden, besonders bevorzugt in Gegenwart von Natriumhydroxid, Kaliumhydroxid oder Cäsiumhydroxid, insbesondere in Gegenwart von Cäsiumhydroxid durchgeführt.

Als Alkin werden Verbindungen der allgemeinen Formel (III) 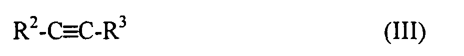 eingesetzt mit der vorstehenden Bedeutung für R² und R³.

Insbesondere können neben Acetylen und Phenylacetylen auch fünktionalisierte Alkine wie Propargylamin eingesetzt werden.

Das erfindungsgemäße Katalysatorsystem wird, bezogen auf die Alkaliverbindung, vorzugsweise CsOH, und die mit dem Alkin umzusetzende Verbindung, vorzugsweise in einer Menge von 0,1 bis 100, besonders bevorzugt 0,1 bis 50, insbesondere 0,1 bis 25 mol-% eingesetzt.

Die Temperatur bei der Umsetzung beträgt vorzugsweise -78 bis 200°C, besonders bevorzugt 0 bis 160°C, insbesondere 80 bis 120°C.

Der Druck im Reaktionssystem beträgt vorzugsweise 1 bis 25 bar (abs), besonders bevorzugt 1 bis 20 bar, insbesondere 1 bis 14 bar. Vorzugsweise wird dabei das Alkin kontinuierlich nachgepreßt.

Sofern mit einem Lösungsmittel gearbeitet wird, beträgt der Anteil an Lösungsmittel, bezogen auf die mit dem Alkin umzusetzende Verbindung, vorzugsweise 2 bis 99 Gew.-%, besonders bevorzugt 20 bis 80 Gew.-%.

Die Umsetzung kann kontinuierlich oder diskontinuierlich in allen dafür geeigneten Reaktoren durchgeführt werden. Die Reaktionszeit beträgt in der Regel 0,01 bis 48 Stunden, vorzugsweise 0,1 bis 12 Stunden, insbesondere 0,1 bis 6 Stunden.

Die Erfindung wird durch die nachfolgenden Beispiele näher erläutert, wobei mit* angegebene Ausbeuten nicht optimiert sind:

### Beispiele

### CsOH · H₂O-katalysierte Vinylierung

### Verfahren C: Umsetzung mit Phenylacetylen (1a)

### z.B. Herstellung von 2-Methyl-2,4-diphenyl-3-butennitril (5a):

### Beispiel 1

In einem 10 ml fassenden Schlenk-Kolben wurde CsOH · H₂O (12 mg, 72 µmol) vorgelegt. Sodann wurden NMP (1 ml), 1-Phenylethylcyanid (4a) (50 µl, 0,38 mmol) und Phenylacetylen (80 µl, 0,76 mmol) nacheinander mit einer Spritze bei -78°C zugegeben. Das Reaktionsgemisch wurde für fünf Minuten stark gerührt. Sodann wurde das Reaktionsgemisch in Ether (30 ml) gegossen und mit Wasser (2 x 20 ml) und Kochsalzlösung (10 ml) gewaschen. Die Etherphase wurde getrocknet (MgSO₄). Nach Verdampfen des Lösungsmittels wurde das Produkt durch Flashchromatographie (Pentan/Ethylacetat 9:1) gereinigt, wobei das gewünschte Produkt (5a) als farbloses Öl (68 mg, 83 %) erhalten wurde. Es wurde ein cis:trans-Verhältnis von 87:13 erhalten.

### Verfahren D: Umsetzung mit Acetylen:

### z.B. Herstellung von 2-Methyl-2-phenyl-3-butennitril (5c):

### Beispiel 2

In einem 50 ml fassenden Schlenk-Kolben wurde CsOH · H₂O (254 mg, 1,5 mmol) mit NMP (20 ml) und 1-Phenylethylcyanid (4a) (1 g, 7,6 mmol) vorgelegt. Das Reaktionsgemisch wurde stark gerührt, und Acetylen wurde für 1,5 Stunden langsam durch die Lösung geleitet. Nach der wie in Verfahren C beschriebenen Aufarbeitung wurde das Produkt (5c) als farbloses Öl (955 mg, 80 %) erhalten.

### Beispiel 3

Ausgehend von 1-Phenylpropylcyanid wurde 2-Ethyl-2,4-diphenyl-3-butennitril (5b) in einer Ausbeute von 78 % erhalten. das cis:trans-Verhältnis betrug 85:15.

### Verfahren E: Umsetzung von Stickstoffheterocyclen mit Phenylacetylen

### Umsetzung von Pyrrolen mit Phenylacetylen:

Ein 25 ml Schlenk-Rohr wurde mit CsOH · H₂O (306 mg, 1,8 mmol) gefüllt. Nacheinander wurden NMP (5 ml), Pyrrol (0,82 ml, 12,2 mmol) und Phenylacetylen (1 ml, 9,12 mmol) mit einer Spritze zugegeben und 36 h bei 90°C gerührt. Das Reaktionsgemisch wurde in Ether (2 x 200 ml) aufgenommen und mit H₂O (2 x 50 ml) und gesättigter NaCl-Lösung (50 ml) extrahiert. Die etherische Phase wurde über MgSO₄ getrocknet und das Lösungsmittel in Vakuum entfernt. Das Produkt wurde durch Säulen-Chromatographie (Pentan/Ethylacetat 98,2) gereinigt und als Öl erhalten (1,22 g, 79 %).

### Umsetzung von Imidazol mit Phenylacetylen:

Ein 25 ml Schlenk-Rohr wurde mit CsOH H₂O (300 mg, 1,8 mmol) gefüllt. Nacheinander wurden mit einer Spritze NMP (5 ml), Imidazol (650 mg, 9,6 mmol) zugegeben. Die Reaktionsmischung wurde heftig gerührt, Phenylacetylen 929 mg, 9,1 mmol) langsam zugetropft und 24 h auf 120°C erhitzt. Die Lösung wurde in Ether (200 ml) gegossen, mit Wasser (4 x 50 ml) extrahiert und über MgSO₄ getrocknet. Das Produkt wurde durch Rekristallisation aus Ether als Nadeln (1,28 g, 83 %, mp = 92 - 94°C) erhalten.

Die Ergebnisse für unterschiedliche Temperaturen, Katalysatormengen und Stickstoffheterocyclen sind in der nachstehenden Tabelle I angegeben.

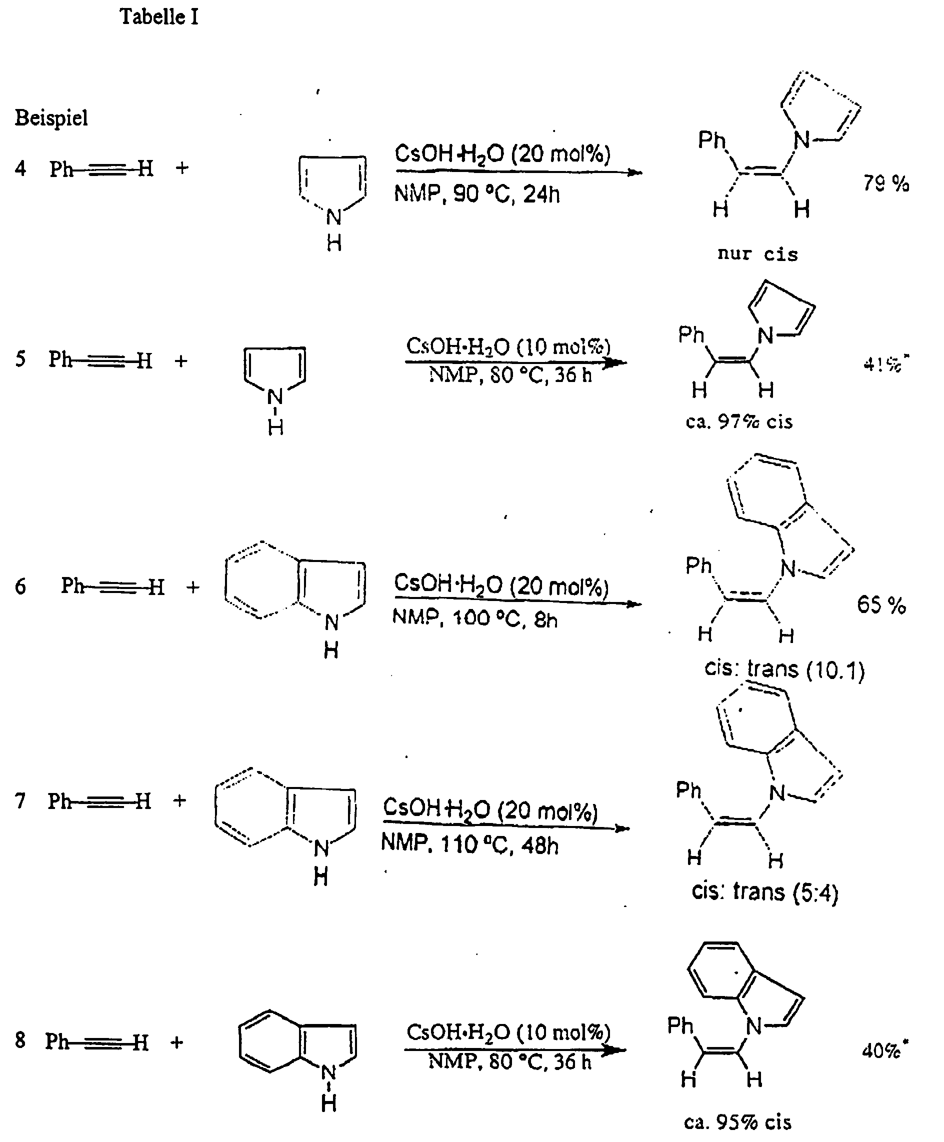

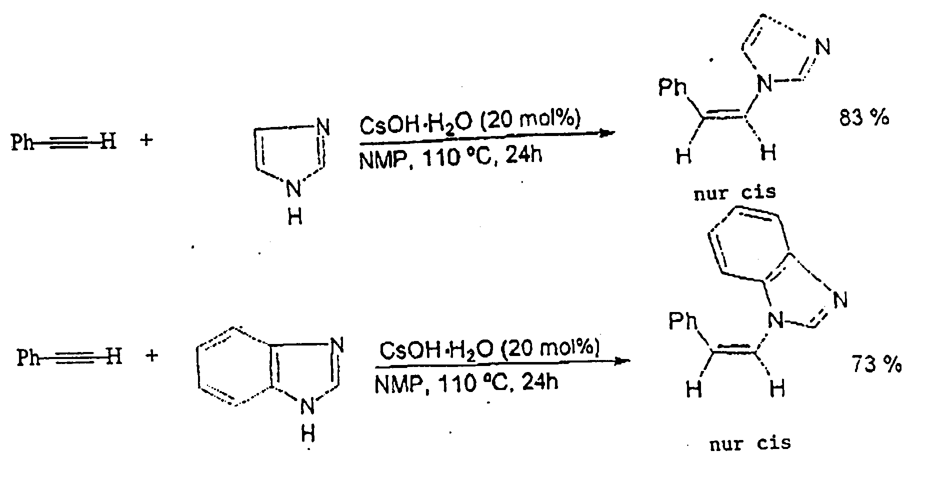

### Verfahren F: Umsetzung von sekundären Aminen z.B. Diphenylamin mit Phenylacetylen

### Beispiel 11

Ein 25 ml Schlenk-Rohr wurde mit CsOH · H₂O (300 mg, 1,8 mmol) und Diphenylamin (1,54, 9,12 mmol) gefüllt. Nacheinander wurden NMP (5 ml) und Phenylacetylen (1 ml, 9,13 mmol) mit einer Spritze zugegeben und 3 h bei 90°C gerührt. Das Reaktionsgemisch wurde in Ether (2 x 200 ml) aufgenommen und mit H₂O (2 x 30 ml) und gesättigter NaCl-Lösung (50 ml) extrahiert. Die etherische Phase wurde über MgSO₄ getrocknet und das Lösungsmittel im Vakuum entfernt. Das Produkt wurde durch Rekristallisation (Ether/Pentan) gereinigt (2,04 g, 82 %).

### Verfahren G: Umsetzung von Phenylacetylen mit Alkoholen z.B. Methanol

Ein 25 ml Schlenk-Rohr wurde mit CsOH · H₂O (336 mg, 2,0 mmol) gefüllt. Anschließend wurden nacheinander mit einer Spritze NMP (10 ml) Methanol (0,6 ml, 14,7 mmol) und Phenylacetylen (1,00 g, 9,8 mmol) zugegeben und die Reaktionsmischung für 12 h auf 100°C erhitzt. Nach Abkühlen auf Raumtemperatur wurde die Lösung in gesättigte NaCl-Lösung gegossen und dreimal mit 25 ml Et₂O extrahiert. Die vereinigten Et₂O-Phasen wurden mit Wasser (3 x 25 ml) gewaschen und über MgSO₄ getrocknet. Nach Entfernung des Lösungsmittels im Vakuum wurde das gewünschte Produkt durch Flash-Chromatographie auf basischem Aluminiumoxid (Pentane/Ethylacetat 19,1) als farbloses Öl (1,18 g, 90 %) erhalten.

Die Ergebnisse für unterschiedliche Alkohole sind in der nachstehenden Tabelle II zusammengefaßt.

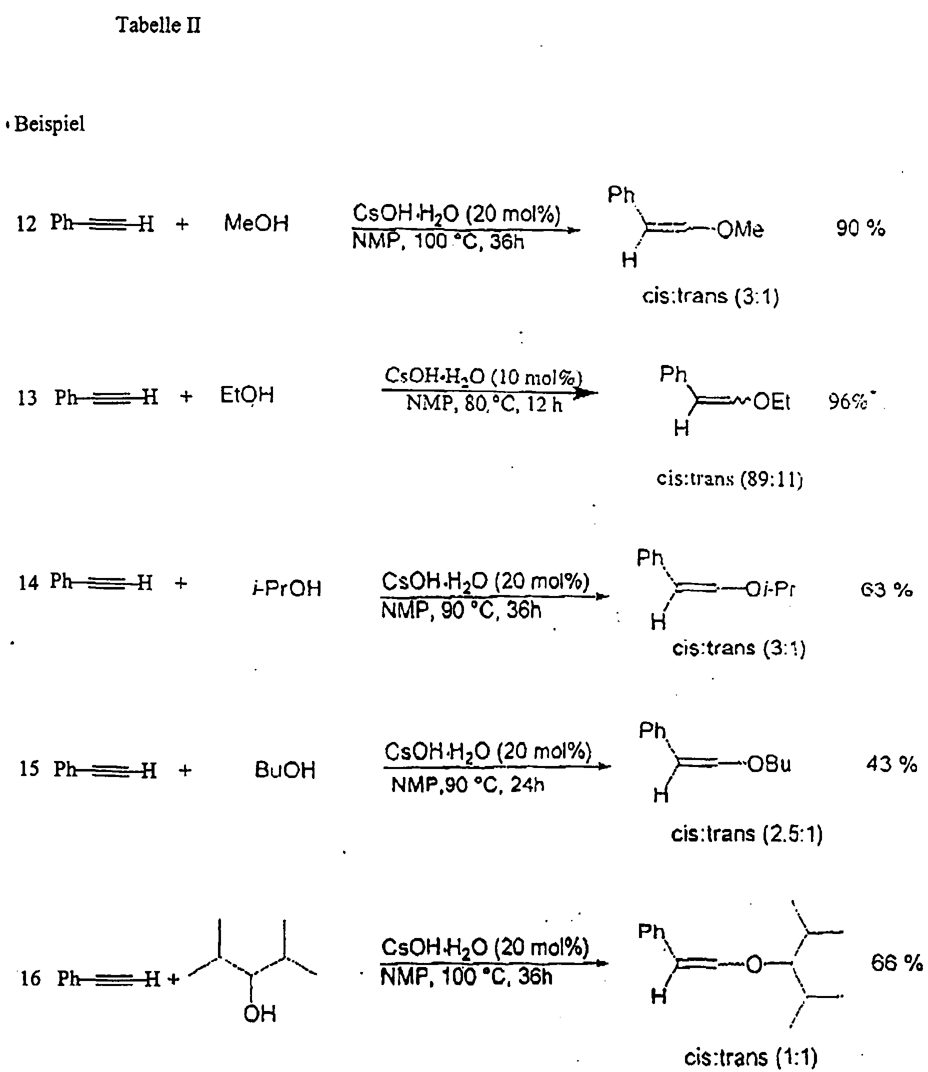

### Beispiel 17

### Alkenylierung von Arylalkynitril im System NMP/KOH:

In einem 250 ml Vierhalskolben werden 100 ml NMP und 2,2 g (0,04 mol) KOH gegeben. Die Suspension wird mit Acetylen gesättigt und auf 0°C gekühlt. Bei gleichzeitigem Einleiten von Acetylen werden 24 g (0,2 mol) Phenylacetonitril bei 0°C zugegeben. Acetylen wird 4 h lang in die Reaktionsmischung eingegast. Zur Aufarbeitung werden 50 ml MTBE und 50 ml H₂O in den Kolben gegeben. Nach der Phasentrennung wird die wäßrige Phase neutralisiert und erneut mit MTBE extrahiert. Die vereinigten organischen Phasen werden eingeengt. Die Ausbeute an Phenylvinylacetonitril beträgt 49 %.

## Patentansprüche

1. Verwendung von CsOH · H₂O als Katalysator bei der Alkenylierung von organischen Verbindungen, die Gruppen -NH, -OH oder >CH-Z
mit Z = CN, COOR
mit R = H oder C₁₋₆-Alkyl
enthalten, mit Ausnahme von 1,2-Aminoalkanolen, durch Umsetzung der Gruppen mit organischen Verbindungen, die eine C-C-Dreifachbindung enthalten.

2. Verfahren zur Herstellung von Alken-Verbindungen der allgemeinen Formel (IV) 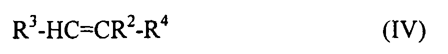 mit der Bedeutung
R², R³ unabhängig H, NH₂ oder C₁₋₂₀-Kohlenwasserstoffrest, der ein- oder mehrfach durch C₁₋₆-Alkyl und/oder Halogen substituiert und/oder von nicht benachbarten Heteroatomen unterbrochen sein und/oder C-C-Doppelbindungen enthalten kann,
R⁴ R⁵R⁶N-, R⁵R⁶C(Z)- oder R⁷-O- mit
R⁵, R⁶, R⁷ unabhängig wie für R³ definiert mit Ausnahme von H, wobei R⁷-O- kein 1,2-Aminoalkanolrest sein kann,
Z CN, COOR mit R = H oder C₁₋₆-Alkyl
durch Umsetzung von Verbindungen der allgemeinen Formel (V) 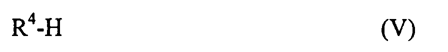 ausgenommen 1,2-Aminoalkanole,
mit Verbindungen der allgemeinen Formel (III) 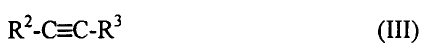 in Gegenwart eines basischen Katalysators, dadurch gekennzeichnet, daß als basischer Katalysator CsOH · H₂O eingesetzt wird.

3. Verfahren zur Herstellung von Alken-Verbindungen der allgemeinen Formel (IV) 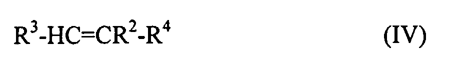 mit der Bedeutung
R², R³ wie in Anspruch 2 definiert,
R⁴ R⁵R⁶N-, R⁵R⁶C(CN)- oder R⁷-O- mit
R⁵, R⁶, R⁷ unabhängig wie für R³ definiert mit Ausnahme von H,
Z wie in Anspruch 2 definiert,
durch Umsetzung von Verbindungen der allgemeinen Formel (V) 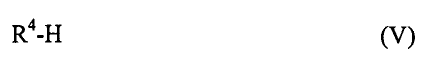 mit Verbindungen der allgemeinen Formel (III) 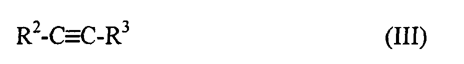 in Gegenwart eines basischen Katalysators, dadurch gekennzeichnet, daß die Umsetzung in einem Lösungsmittel auf Basis von NMP durchgeführt wird.

4. Verfahren nach einem der Ansprüche 2 oder 3, dadurch gekennzeichnet, daß bei Umsetzung in einem Lösungsmittel auf Basis von NMP als basischer Katalysator ein Alkalihydroxid eingesetzt wird.

5. Verfahren nach Anspruch 4, dadurch gekennzeichnet, daß als basischer Katalysator CsOH · H₂O eingesetzt wird.
